(19) Europäisches Patentamt
European Patent Office
Office européen des brevets

(11) **EP 0 848 064 B1**

(12) # EUROPÄISCHE PATENTSCHRIFT

(45) Veröffentlichungstag und Bekanntmachung des Hinweises auf die Patenterteilung:
**14.11.2001 Patentblatt 2001/46**

(51) Int Cl.[7]: **C12P 21/02**, C07K 7/08, C12N 1/20, A61K 38/10 // (C12P21/02, C12R1:465)

(21) Anmeldenummer: **97121438.2**

(22) Anmeldetag: **05.12.1997**

(54) **Neues Antibiotikum, Feglymycin, Verfahren zu seiner Herstellung und Verwendung**

New antibiotic, Feglymycine, process for its preparation and use of the same

Substance antibiotique, Feglymycine, sa préparation et son utilisation

(84) Benannte Vertragsstaaten:
**AT BE CH DE DK ES FI FR GB GR IE IT LI LU NL PT SE**

(30) Priorität: **13.12.1996 DE 19652008**

(43) Veröffentlichungstag der Anmeldung:
**17.06.1998 Patentblatt 1998/25**

(73) Patentinhaber: **HOECHST AKTIENGESELLSCHAFT**
**65929 Frankfurt am Main (DE)**

(72) Erfinder:
- **Vértesy, Laszlo Dr.**
  **65817 Eppstein (DE)**
- **Knauf, Martin Dr.**
  **60528 Frankfurt (DE)**
- **Wink, Joachim Dr.**
  **63322 Rödermark (DE)**
- **Isert, Dieter Dr.**
  **65760 Eschborn (DE)**
- **Stahl, Wilhelm Dr.**
  **65510 Idstein (DE)**
- **Riess, Günther Dr.**
  **65795 Hattersheim (DE)**
- **Aszodi, Jozsef Dr.**
  **77340 Pontault Combault (FR)**
- **Le Beller, Dominique Dr.**
  **60880 Jaux (FR)**

(56) Entgegenhaltungen:
**EP-A- 0 603 030** **EP-A- 0 603 031**

- **MAKI NISHIO ET AL.: "Siamycins I and II, New Anti-HIV Peptides" J. ANTIBIOT., Bd. 48, Nr. 5, 1995, Seiten 433-434, XP002057234**
- **OLIVIER POTTERAT ET AL.: "Aborycin -a Tricyclic 21-Peptide Antibiotic Isolated from Streptomyces griseoflavus" LIEBIGS ANN. CHEM., Bd. 7, 1994, Seiten 741-743, XP002057235**

EP 0 848 064 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft das neue Antibiotikum Feglymycin, Verfahren zu seiner Herstellung und Verwendung.

Zur Behandlung von bakteriellen Infektionskrankheiten wird eine große Zahl von Antibiotika therapeutisch eingesetzt. Die Krankheitserreger werden aber zunehmend resistent gegen die verwendeten Arzneimittel, es droht sogar eine große Gefahr durch sogenannte multiresistente Keime, die nicht nur gegen einzelne Antibiotikagruppen, wie z. B. β-Lactam-Antibiotika oder Glycopeptide oder Macrolide wiederstandsfähig geworden sind, sonderen gleichzeitig mehrere Resistenzen tragen. Es gibt sogar Krankheitserreger, die gegen alle im Handel erhältlichen Antibiotika resistent geworden sind, Infektionskrankheiten, die durch solche Keime verursacht werden, sind nicht mehr therapierbar. Deshalb gibt es einen großen Bedarf an neuen Mitteln, die gegen resistente Keime eingesetzt werden können. Es sind zwar in der Literatur viele Tausend Antibiotika beschrieben worden, die meisten sind jedoch zu toxisch um als Arzneimittel eingesetzt werden zu können.

Die Resistenzentwicklung ist auch das Problem bei der Bekämpfung der Immunschwäche, der sogenannten AIDS-Erkrankung, die vom neuen Virus-Typ, dem "Human Immunodeficiency Virus" (HIV) verursacht wird. Es gibt noch kein Medikament zur Heilung einer AIDS-Erkrankung. Die Mittel, die bis jetzt eingesetzt worden sind, können zwar die Lebenserwartung von HIV-infizierten Personen verlängern, durch die Bildung von resistenten Viren ist die Medizin jedoch auf neuartige, untoxische Virostatika dringend angewiesen.

Es ist überraschend gefunden worden, daß die Streptomyces spec. HAG 4675, DSM 11 171 ein neues Antibiotikum, das Feglymycin, zu bilden vermag, das nicht nur antibakteriell wirksam und gut verträglich ist, sondern auch die "Human Immunodeficiency"-Viren wirksam hemmt.

Erfindungsgegenstand ist demzufolge die Verbindung Feglymycin sowie seine physiologisch verträglichen Salze sowie seine offensichtlichen chemischen Äquivalente.

Feglymycin, Summenformel: $C_{95}H_{97}N_{13}O_{30}$, MG 1900,90.

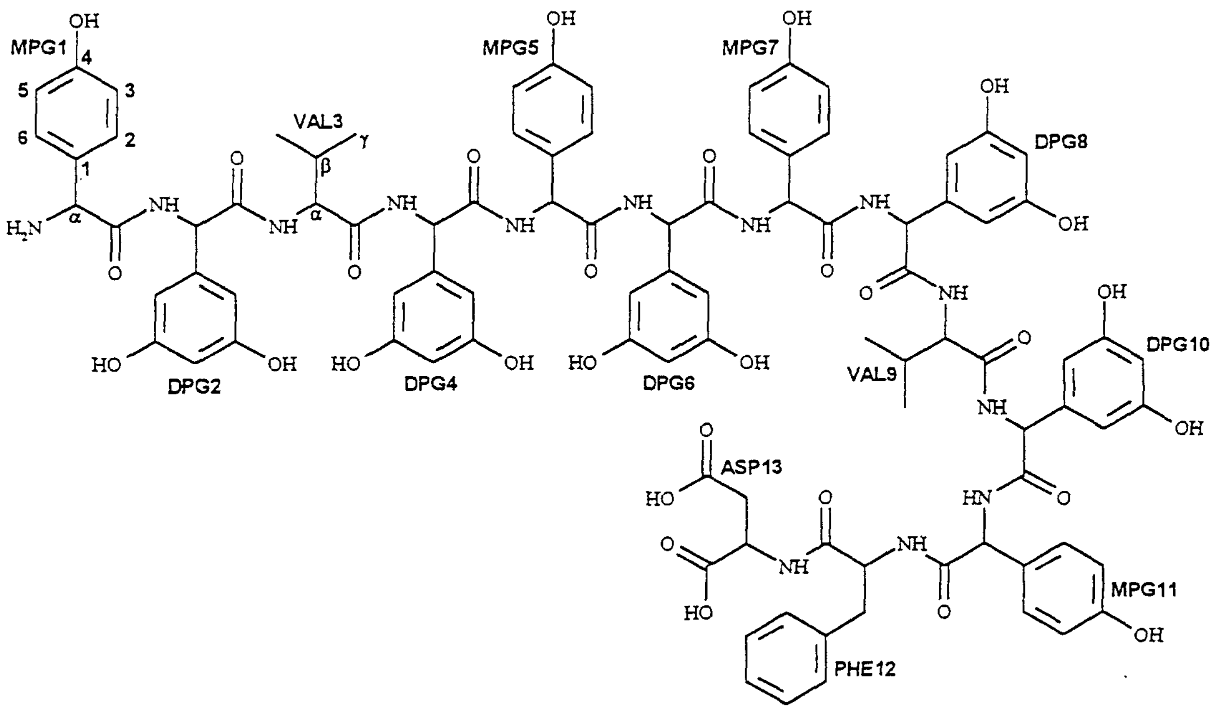

worin die Abkürzungen die folgenden Bedeutungen haben:
MPG = Monhydroxyphenylglycin, DPG = Dihydroxyphenylglycin, VAL = Valin,
PHE = Phenylalanin, ASP = Asparaginsäure

Durch die angegebenen Struktur- bzw. Summenformeln unterscheidet sich das Antibiotikum Feglymycin von literaturbekannten Substanzen. Die erfindungsgemäße Verbindung weist ein charakteristisches Ultraviolett-Spektrum auf.

Weiterhin gehören zum Gegenstand der vorliegenden Erfindung die Verfahren zur Herstellung der genannten

Verbindungen. Ein Verfahren zur Herstellung der genannten Verbindungen ist dadurch gekennzeichnet, daß der Mikroorganismus Streptomyces species HAG 4675 (DSM 11171) in einem wäßrigen Nährmedium kultiviert wird und die Zielverbindungen anschließend isoliert und gereinigt werden. Der genannte Mikroorganismus ist am 24. September 1996 unter den Bedingungen des Budapester Vertrags bei der Deutschen Sammlung von Mikroorganismen und Zellkulturen, Mascheroder Weg 1b, D-38124 Braunschweig unter der Nummer DSM 11171 hinterlegt worden.

Streptomyces spec. DSM 11171 besitzt weißes Luftmycel und gelbe Sporenketten. Er bildet die für die Streptomyceten charakteristischen Sporenketten in Spiralen. Die Zellwand enthält L,L Diaminopimelinsäure als charakteristische Aminosäure und Glucose sowie Mannose als Zucker, dies sind charakteristische Merkmale für die Gattung Streptomyces. In einer Nährlösung, die eine Kohlenstoffquelle und eine Stickstoffquelle sowie die üblichen anorganischen Salze enthält, produziert der Streptomyces spec. DSM 11171 die Verbindung Feglymycin.

Anstelle des Stammes DSM 11171 können auch dessen Mutanten und Varianten eingesetzt werden, soweit sie die erfindungsgemäßen Verbindungen synthetisieren. Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethansulfonat (EMS); 2-Hydroxy-4-methoxy-benzophenon (MOB) oder N-Methyl-N'-nitro-N-nitrosoguanidin (MNNG) erzeugt werden.

Das Screening nach Mutanten und Varianten, die das erfindungsgemäße Antibiotikum produzieren, kann durch Bestimmung der biologischen Aktivität des in der Kulturbrühe angehäuften Wirkstoffes, beispielsweise durch Austesten der antibakteriellen Wirkung erfolgen.

Als bevorzugte Kohlenstoffquelle für die aerobe Fermentation eignen sich assimilierbare Kohlenhydrate und Zuckeralkohole, wie Glucose, Laktose oder D-Mannit sowie kohlenhydrathaltige Naturprodukte, wie z.B. Malzextrakt. Als stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Hafer, Soja oder der Baumwollpflanze, Destillationsrückstände der Alkoholherstellung, Fleischmehle oder Hefeextrakte, aber auch Ammoniumsalze und Nitrate. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung des Feglymycins verläuft besonders gut z. B. in einer Nährlösung, die etwa 0,5 bis 5 % Haferflocken, vorzugsweise 1 bis 2 % und eine Spurenelementlösung in einer Konzentration von 0,1 bis 0,5 % vorzugsweise 0,2 bis 0,3 % enthält. Die Spurenelementlösung enthält $CaCl_2$, Fe III Citrat, $MnSO_4$, $ZnCl_2$, $CuSO_4$, Natriumtetraborat, $CoCl_2$ und Natriummolybdat.

Die Kultivierung erfolgt aerob, also beispielsweise submers unter Schütteln oder Rühren in Schüttelkolben oder Fermentern, gegebenenfalls unter Einführen von Luft oder Sauerstoff. Die Fermentation kann beispielsweise in Steilbrustflaschen oder Rundkolben verschiedener Volumina, in Glasfermentern oder $V_2$A-Stahltanks durchgeführt werden. Sie kann in einem Temperaturbereich von etwa 20 bis 35° C, vorzugsweise bei etwa 25 bis 30° C, durchgeführt werden. Der pH-Wert sollte zwischen 4 und 10 liegen, vorteilhaft zwischen 5,5 und 8,5. Man kultiviert den Mikroorganismus unter diesen Bedingungen im allgemeinen über einen Zeitraum von 20 bis 300 Stunden, bevorzugt 24 bis 140 Stunden. Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z. B. indem man ein versportes Mycel in eine Nährlösung überimpft und etwa 20 bis 120 Stunden, bevorzugt 24 bis 72 Stunden, wachsen läßt. Das versporte Mycel kann beispielsweise erhalten werden, indem man den Stamm etwa 1 bis 40 Tage, bevorzugt 3 bis 10 Tage, auf einem festen oder flüssigen Nährboden, beispielsweise Hefe-Malz-Agar oder Kartoffel-Dextrose-Agar, wachsen läßt.

Der Fermentationsverlauf und die Bildung des Antibiotikums Feglymycin kann entsprechend dem Fachmann bekannten Methoden, wie z. B. durch Austestung der biologischen Aktivität in Bioassays oder durch chromatographische Methoden wie Dünnschichtchromatographie (DC) oder Hochleistungsflüssigkeitschromatographie (HPLC) verfolgt werden.

Das Antibiotikum Feglymycin kann sowohl im Mycel als auch im Kulturfiltrat vorkommen, gewöhnlich befindet sich die Hauptmenge in der Zellmasse (Mycel). Es ist deshalb zweckmäßig, diese durch Filtration oder Zentrifugation vom Filtrat zu trennen. Das Filtrat wird mit einem Adsorptionsharz als feste Phase extrahiert. Das Mycel wird zweckmäßigerweise mit Methanol oder Aceton extrahiert, es können aber auch andere Lösungsmittel verwendet werden.

Die Extraktionen können in einem weiten pH-Bereich durchgeführt werden, es ist jedoch zweckmäßig, im neutralen oder schwach sauren Milieu, vorzugsweise zwischen pH 3 und pH 7 zu arbeiten. Die Extrakte können z. B. im Vakuum konzentriert und getrocknet werden.

Eine Methode der Isolierung des Feglymycins ist die Lösungsverteilung in an sich bekannter Weise.

Eine andere Methode der Reinigung ist die Chromatographie an Adsorptionsharzen wie z. B. an Diaion® HP-20 (Mitsubishi Casei Corp., Tokyo), an Amberlite® XAD 7 (Rohm and Haas, USA), an Amberchrom® CG, (Toso Haas, Philadelphia, USA) oder an ähnlichen. Geeignet sind darüber hinaus zahlreiche Reverse Phase-Träger, z. B. $RP_{18}$, wie sie z. B. im Rahmen der Hochdruckflüssigkeits-Chromatographie (HPLC) allgemein bekannt geworden sind.

Eine weitere Reinigungsmöglichkeit für die erfindungsgemäßen Antibiotika besteht in der Verwendung von

sog. Normal-Phasen-Chromatographie-Trägern, wie z. B. Kieselgel oder $Al_2O_3$ oder anderen in an sich bekannter Weise.

Ein alternatives Isolierungsverfahren ist die Verwendung von Molekularsieben, wie z. B. Fractogel® TSK HW-40, Sephadex® G-25 und andere, in an sich bekannter Weise. Es ist darüber hinaus auch möglich, aus angereichertem Material das Feglymycin durch Kristallisation zu gewinnen. Geeignet hierzu sind z. B. organische Lösungsmittel und ihre Gemische, wasserfrei oder mit Wasserzusatz. Ein zusätzliches Verfahren zur Isolierung und Reinigung der erfindungsgemäßen Antibiotika besteht in der Verwendung von Anionenaustauschern, vorzugsweise im pH-Bereich von 4 bis 10 und Kationenaustauschern vorzugsweise im pH-Bereich von 2 bis 5. Besonders geeignet ist hierfür die Verwendung von Pufferlösungen, denen man Anteile von organischen Lösungsmitteln hinzugefügt hat.

Das Antibiotikum Feglymycin oder abgeleitete chemische Derivate können nach dem Fachmann bekannten Methoden in die entsprechenden pharmakologisch verträglichen Salze übergeführt werden.

Offensichtliche chemische Äquivalente der erfindungsgemäßen Verbindungen sind Verbindungen die einen geringfügigen chemischen Unterschied aufweisen, also die gleiche Wirksamkeit haben, oder sich unter milden Bedingungen in die erfindungsgemäßen Verbindungen umwandeln. Zu den genannten Äquivalenten gehören z.B. Ester, Aminoderivate, Komplexe oder Addukte der bzw. mit den erfindungsgemäßen Verbindungen.

Unter pharmakologisch verträglichen Salzender erfindungsgemäßen Verbindungen versteht man sowohl anorganische als auch organische Salze, wie sie in Remington's Pharmaceutical Sciences (17. Auflage, Seite 1418 (1985)) beschrieben sind. Als Salze kommen insbesondere Alkali-, Ammonium-, Erdalkalisalze, Salze mit physiologisch verträglichen Aminen und Salze mit anorganischen oder organischen Säuren wie z. B. HCl, HBr, $H_2SO_4$, Maleinsäure, Fumarsäure in Frage.

Die physikalisch-chemischen sowie spektroskopischen Eigenschaften der erfindungsgemäßen Antibiotika lassen sich wie folgt zusammenfassen:

Feglymycin:

Aussehen:

farblose, in Methanol, Acetonitril und Wasser lösliche Substanz. Stabil in neutralem und mild alkalischem Milieu, jedoch unbeständig in stark-saurer und stark-alkalischer Lösung.

| | |
|---|---|
| Summenformel: | $C_{95}H_{97}N_{13}O_{30}$ |
| Molekulargewicht: | 1900,90 |
| $^1$H-NMR: | siehe Tabelle 1 |
| V-Maxima (log ε): | 280 nm (4.16) |

$[\alpha]_D^{20}$ = -106 °

Tab.1:

| $^1$H- und $^{13}$C-chemische Verschiebungen von Feglymycin | | | |
|---|---|---|---|
| **Sequenz-Nr.** | **Atomposition** | **$^1$H-chem. Verschiebung** | **$^{13}$C-chem. Verschiebung** |
| MPG 1 | $NH_2$ | n.d. | -- |
| | αH | 4.982 (b) | 54.559 (2) |
| | 1 | -- | 123.917 |
| | 2,6 | 7.341 (m) | 129.170 (12) |
| | 3,5 | 6.807 (d) | 115.257 (4) |
| | 4 | -- | 157.870 |
| | OH | 9.730 | -- |
| | CO | 166.568 | -- |
| DPG2 | NH | 8.928 | -- |
| | αH | 5.492 | 56.157 |
| | 1 | -- | 140.440 |

Tab.1: (fortgesetzt)

| $^{1}H$- und $^{13}C$-chemische Verschiebungen von Feglymycin | | | |
|---|---|---|---|
| **Sequenz-Nr.** | **Atomposition** | **$^{1}H$-chem. Verschiebung** | **$^{13}C$-chem. Verschiebung** |
| | 2,6 | 6.365 | 105.445 |
| | 3,5 | -- | 157,741 |
| | 4 | 6.130 | 101.589 |
| | OH | 9.220 | -- |
| | CO | -- | 168.603 |
| VAL3 | NH | 7.960 | -- |
| | αH | 4.360 | 56.789 |
| | βH | 1.875 | 31.240 |
| | γH | 0.546 | 18.860 |
| | γH | 0.524 | 16.951 |
| | CO | -- | 169.798 |
| DPG4 | NH | 8.515 | -- |
| | αH | 5.532 | 55.515 |
| | 1 | -- | 140.647 |
| | 2,6 | 6.253 | 105.197 |
| | 3,5 | -- | 157.573 |
| | 4 | 6.067 | 101.325 |
| | OH | 9.140 | -- |
| | CO | -- | 168.591 |
| MPG5 | NH | 8.651 | -- |
| | αH | 5.611 | 54.680 |
| | 1 | -- | 128.298 |
| | 2,6 | 7.065 | 127.775 |
| | 3,5 | 6.554 | 114.530 |
| | 4 | -- | 156.180 |
| | OH | 9.281 | -- |
| | CO | -- | 169.197 |
| DPG6 | NH | 8.774 | -- |
| | αH | 5.502 | 55.519 |
| | 1 | -- | 140.232 |
| | 2,6 | 6.169 | 105.146 |
| | 3,5 | -- | 157.598 |
| | 4 | 6.049 | 101.325 |
| | OH | 9.086 | -- |
| | CO | -- | 168.591 |
| MPG7 | NH | 8.676 | - |
| | αH | 5.590 | 54.779 |

Tab.1: (fortgesetzt)

| $^1H$- und $^{13}C$-chemische Verschiebungen von Feglymycin | | | |
|---|---|---|---|
| **Sequenz-Nr.** | **Atomposition** | **$^1H$-chem. Verschiebung** | **$^{13}C$-chem. Verschiebung** |
| | 1 | -- | 128.298 |
| | 2,6 | 7.051 | 127.775 |
| | 3,5 | 6.576 | 114.530 |
| | 4 | -- | 156.180 |
| | OH | 9.302 | -- |
| | CO | -- | 169.113 |
| DPG8 | NH | 8.631 | -- |
| | αH | 5.400 | 55.893 |
| | 1 | -- | 140.666 |
| | 2,6 | 6.200 | 105.086 |
| | 3,5 | -- | 157.590 |
| | 4 | 6.047 | 101.325 |
| | OH | 9.085 | -- |
| | CO | -- | 168.983 |
| VAL9 | NH | 8.126 | -- |
| | αH | 4.364 | 56.552 |
| | βH | 1.881 | 31.240 |
| | γH | 0.609 | 17.387 |
| | γH | 0.588 | 18.886 |
| DPG10 | NH | 8.478 | -- |
| | αH | 5.458 | 55.503 |
| | 1 | -- | 140.559 |
| | 2,6 | 6.290 | 105.428 |
| | 3,5 | -- | 157.695 |
| | 4 | 6.084 | 101.393 |
| | OH | 9.154 | -- |
| | CO | -- | 168.600 |
| MPG11 | NH | 8.519 | -- |
| | αH | 5.300 | 55.231 |
| | 1 | -- | 128.050 |
| | 2,6 | 6.933 | 127.971 |
| | 3,5 | 6.567 | 114.530 |
| | 4 | -- | 156.180 |
| | OH | 9.320 | -- |
| | CO | -- | 169.216 |
| PHE12 | NH | 8.362 | -- |
| | αH | 4.523 | 53.541 |

Tab.1: (fortgesetzt)

| $^1H$- und $^{13}C$-chemische Verschiebungen von Feglymycin | | | |
|---|---|---|---|
| **Sequenz-Nr.** | **Atomposition** | **$^1H$-chem. Verschiebung** | **$^{13}C$-chem. Verschiebung** |
| | βH | 3.038/2.811 | 37.270 |
| | 1 | -- | 137.191 |
| | 2,6 | 7.238 | 129.097 |
| | 3,5 | 7.222 | 127.824 |
| | 4 | 7.160 | 126.021 |
| | CO | - | 169.949 |
| ASP13 | NH | 8.224 | -- |
| | αH | 4.433 | 48.440 |
| | βH | 2.575/2.492 | 35.927 |
| | COOH§ | 12.45 | -- |
| | COOH§ | 12.70 | -- |
| | COOH§ | -- | 171.300 |
| | COOH§ | -- | 171.941 |

§: Die Zuordnung, welche der Carboxylgruppen sich in α- oder β-Position befindet, konnte nicht getroffen werden

Es wurde weiterhin gefunden, daß die erfindungsgemäße Verbindung antibakterielle Wirkungen aufweist. Tabelle 2 faßt die Minimalen Hemmkonzentrationen von Feglymycin beispielhaft zusammen.

Tabelle 2

| In-Vitro Aktivität des Feglymycins gegen grampositive Bakterien im Reihenverdünnungstest. | |
|---|---|
| Minimale Hemmkonzentration in µg/ml | |
| Keim | (µg/ml) |
| *S. aureus* SG 511 | 64 |
| *S. aureus* 285 | 64 |
| *S. aureus* 503 | 32 |
| *S. aureus* FH 1982 | 64 |
| *S. aureus* 701E | 64 |
| *S. aureus* 9 Tüb | 64 |
| *S. aureus* 8236 | 64 |
| *S. epidermidis* ZH 2c | 64 |
| *S. epidermidis* 763 | 64 |
| *S. epidermidis* 799 | 64 |
| *S. pyogenes* 308A | 32 |
| *S. pyogenes* 77A | 32 |

Es ist darüber hinaus völlig überraschend gefunden worden, daß Feglymycin eine Hemmwirkung auf Human Immundeficiency Viren zeigt. Viele Wirkstoffe hemmen das Virus oder virale Enzyme nur dann, wenn diese in isolierter Form vorliegen. In Zellkulturen oder gar in lebenden Organismen, wie z. B. in Menschen und Tieren, sind solche Agenzien häufig inaktiv, da sie die Zellwand der Zellen, in denen sich die Viren befinden und vermehren, nicht zu durchdringen vermögen. Feglymycin ist jedoch im Zellkultur assay gut wirksam. Der $IC_{50}$ beträgt weniger als 7 µg pro mL. Die Hemmwirkung kann durch lichtmikroskopische Beobachtung von HIV-typischen Syncytien gemessen werden. Die Reduktion der Syncytienbildung ist hierbei ein Maß für die Wirksamkeit des Agenzes, in diesem Fall des Feglymycins. Man kann aber auch die Aktivität des Feglymycins durch Messen der Menge der HIV-Partikel in Zellkulturüberständen z. B. mittels kommerziell erhältlichen p24 Antigen-Tests bestimmen.

Die Verträglichkeit des Feglymycins ist in der wirksamen Konzentration und darüber gut. Cytotoxische Wirkungen oder andere Toxizitäten wurden nicht beobachtet.

Die vorliegende Erfindung betrifft demzufolge auch die Anwendung der erfindungsgemäßen Verbindungen als Arzneimittel, sowie die Verwendung der betreffenden Verbindungen zur Herstellung von Arzneimitteln zur Behandlung und/oder Prophylaxe von bakteriellen und HIV-Infektionen einhergehen. bakteriellen und HIV-Infektionen einhergehen.

Des weiteren betrifft die vorliegende Erfindung Arzneimittel mit einem Gehalt an der erfindungsgemäßen Verbindung.

Die erfindungsgemäßen Arzneimittel können enteral (oral), parenteral (intramuskulär oder intravenös), rektal oder lokal (topisch) angewendet werden. Sie können in Form von Lösungen, Pulvern (Tabletten, Kapseln einschließlich Mikrokapseln), Salben (Cremes oder Gel), oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0.1 - 1000, vorzugsweise 0.2 - 100 mg/kg Körpergewicht verabreicht. Sie werden zweckmäßig in Dosierungseinheiten verabreicht, die mindestens die wirksame Tagesmenge der erfindungsgemäßen Verbindungen, z. B. 30 - 3000, vorzugsweise 50 - 1000 mg enthalten.

Durch die nachfolgenden Ausführungsbeispiele sowie durch den Inhalt der Patentansprüche soll die vorliegende Erfindung näher erläutert werden.

Beispiel 1: Herstellung einer Sporensuspension des Produzentenstammes.

100 ml Nährlösung (20 g Malzextrakt, 2 g Hefeextrakt, 10 Glucose, 0,5 g $(NH_4)_2HPO_4$ in 1 L Leitungswasser, pH-Wert vor der Sterilisation: 6,0) in einem 500 ml sterilen Erlenmeyerkolben werden mit dem Stamm beimpft und 72 Stunden bei 25°C und 140 UpM auf einer rotierenden Schüttelmaschine inkubiert. Anschließend werden 120 ml Kulturflüssigkeit in einem sterilen 500 ml Erlenmeyerkolben mit dem Nährboden Hafermehlinfus, 2,0 g/L, dem zusätzlich 15 g Agar/L zur Verfestigung zugegeben wurde, gleichmäßig verteilt und dekantiert. Die Kulturen werden 10 bis 14 Tage bei 25° C inkubiert. Die nach dieser Zeit entstandenen Sporen eines Kolbens werden mit 500 ml entionisiertem Wasser, das einen Tropfen eines handelsüblichen nichtionischen Tensids (z.B. ® Triton X 100, Fa. Serva) enthält, abgeschwemmt, sofort weiterverwendet oder bei -22° C in 50 % Glycerin oder in 10 % Dimethylsulfoxid bei -140° C aufbewahrt.

Beispiel 2: Herstellung einer Kultur bzw. einer Vorkultur des Produzentenstammes im Erlenmeyerkolben.

Ein steriler 500 ml Erlenmeyerkolben mit 100 ml der im Beispiel 1 beschriebenen Nährlösung wird mit einer auf einem Schrägröhrchen gewachsenen Kultur oder mit 0,2 ml Sporensuspension angeimpft und auf einer Schüttelmaschine im Dunkeln bei 140 UpM und 25° C inkubiert. Die maximale Produktion der Verbindungen der Formel I ist nach ca. 72 Stunden erreicht. Zum Animpfen von 10 und 100 L Fermentern genügt eine 72 Stunden alte Submerskultur (Animpfmenge ca. 5 %) aus der gleichen Nährlösung.

Beispiel 3: Herstellung des Feglymycin.

Ein 10 L Fermenter wird unter folgenden Bedingungen betrieben:

| | | |
|---|---|---|
| Nährmedium | 2 % Haferflocken | |
| | 0,25 % Spurenelemente | |
| | Spurenelemente: | |
| | $CaCl_2$ $2H_2O$ | 0,3 % |
| | Fe III Citrat | 0,1 % |
| | $MnSO_4$ | 0,02 % |
| | $ZnCl_2$ | 0,01 % |
| | $CuSO_4$ 5H20 | 0,002 % |
| | Natiumtetraborat | 0,02 % |
| | $CaCl_2$ $6H_2O$ | 0,001 % |
| | Natriummolybdat | 0,001 % |

(fortgesetzt)

| | |
|---|---|
| Inkubationszeit | 24 oder 48 Stunden |
| Inkubationstemperatur | 28° C |
| Rührergeschwindigkeit | 200 UpM |
| Belüftung | 5 L Luft/min. |

Durch wiederholte Zugabe weniger Tropfen ethanolischer Polyollösung kann die Schaumbildung unterdrückt werden. Das Produktionsmaxiumum wird nach 48 Stunden erreicht.

Beispiel 4: Isolierung des Antibiotikums Feglymycin.

27 L der nach Beispiel 3 gewonnenen Kulturlösung werden abzentrifugiert und die Zellmasse (~ 1,1 L) zweimal mit je 2,2 L Methanol ausgerührt. Die vereinigten Extrakte werden im Vakuum eingeengt, getrocknet und die Trockenmasse mit Diethylether digeriert. Der so gewaschene, entfettete Rückstand (40 g) wird in Wasser gelöst, auf eine 3 L fassende, mit dem Adsorptionsharz MCI Gels CHP20P gefüllte Säule aufgetragen. Säulenmaße:
Breite x Höhe: 11,3 cm x 30 cm. Eluiert wird mit einem Lösungsmittel-Gradienten von 5 % Isopropanol in Wasser bis 50 % Isopropanol und der Säulenausfluß in Fraktionen je 2 L aufgefangen.
Die Feglymycinhaltigen Fraktionen, die durch HPLC-Analysen überprüft werden, werden gesammelt und im Vakuum konzentriert sowie gefriergetrocknet (3,2 g).

Beispiel 5: Hochdruckflüssigkeitschromatographie (HPLC) des Feglymycins .

| | |
|---|---|
| Säule | Nucleosil® 100 - 5 $C_{18}$AB, 250/4. |
| Mobile Phase | 25 % Acetonitril in 0,05 % Trifluoressigsäure. |
| Flußgeschwindigkeit | 1 mL pro Minute |

Detektion durch UV-Absorption bei 210 nm.
Es wurde für Feglymycin die Retentionszeit von 12 Min. 50 Sekunden gefunden.

Beispiel 6: Anreicherung des Feglymycins.

3 g des nach Beispiel 4 gewonnen Produktes werden auf eine 3 Liter fassende mit Fractogel® TSK HW-40 s gefüllte Säule (Breite x Höhe = 10 cm x 50 cm) aufgetragen. Das Laufmittel: 50 % Acetonitril 10 mM Natriumphosphat-Puffer, pH 7,0 wird mit einer Flußrate von 50 ml pro Minute über die Säule gepumpt und der Säulenausfluß fraktioniert (65 ml) aufgefangen. In den Fraktionen 23 bis 27 befindet sich hauptsächlich das Antibiotikum Feglymycin, 270 mg.

Beispiel 7: Endreinigung des Feglymycins.

Das angereicherte Antibiotikum Feglymycin (270 mg), gewonnen nach Beispiel 6, wird auf einer Nucleosil® 12$C_{18}$AB-HPLC-Säule (Breite x Höhe = 3,2 cm x 25 cm) im Gradientenverfahren mit 5 % bis 30 % Acetonitril in 0,05 % Trifluoressigsäure aufgetrennt. Die durch analytische HPLC (siehe Beispiel 5) untersuchten Fraktionen werden entsprechend zusammengefaßt, im Vakuum eingeengt und gefriergetrocknet. Sie ergeben 21 mg Feglymycin in 97 %iger Reinheit.
Durch FAB-Massenspektrometrie ermitteltes Molekulargewicht des Feglymycins:
$M + M^+ = 1900.58$ (Monoisotopischer Peak), 1901.57 (base Peak).

Beispiel 8: Endreinigung durch präparative HPLC im Phosphatpuffer/ Isopropanolsystem.

Verfahren wie in Beispiel 7, jedoch werden als Laufmittel 10 mM Kaliumphosphatpuffer, pH 7 sowie Isopropanol verwendet. Entsalzung der getrennten Komponenten wie im Beispiel 7. 18 mg Feglymycin in 99 %iger Reinheit.

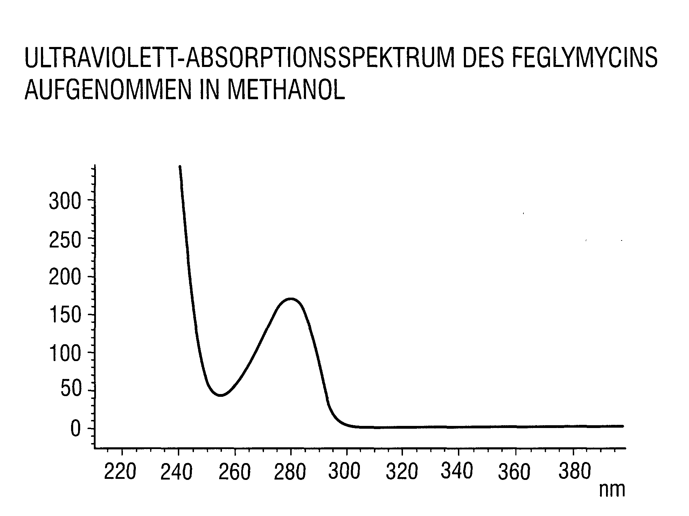

**Patentansprüche**

1. Feglymycin, eine Verbindung der Formel

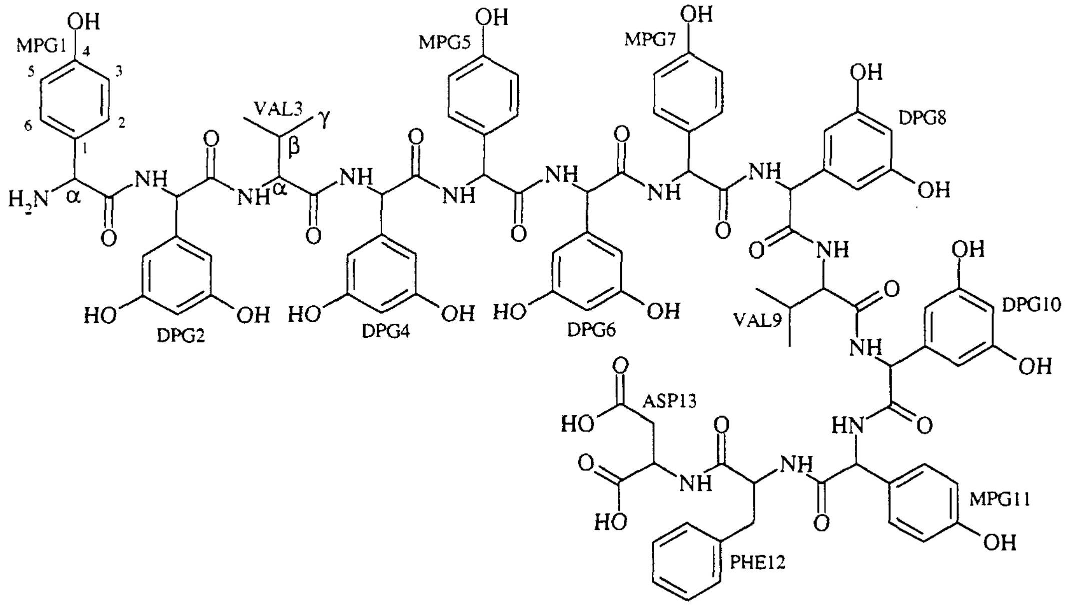

worin die Abkürzungen die folgenden Bedeutungen haben:

MPG = Monohydroxyphenylglycin,
DPG = Dihydroxyphenylglycin,
VAL = Valin, PHE = Phenylalanin,
ASP = Asparaginsäure

deren physiologisch verträgliche Salze sowie deren offensichtliche chemische Äquivalente, die einen geringfügigen chemischen Unterschied bei gleicher Wirksamkeit aufweisen oder sich unter milden Bedingungen in die Verbindung der obigen Formel umwandeln.

2. Verbindung gemäß Anspruch 1, herstellbar indem der Mikroorganismus DSM 11171 bzw. eine seiner Varianten oder Mutanten unter geeigneten Bedingungen fermentiert wird, Feglymycin isoliert wird und ggf. in seine Salze oder chemischen Äquivalente überführt wird.

3. Verfahren zur Herstellung der Verbindung gemäß Anspruch 1, **dadurch gekennzeichnet, daß** der Mikroorganismus DSM 11171 bzw. eine seiner Varianten oder Mutanten unter geeigneten Bedingungen fermentiert wird, Feglymycin isoliert wird und ggf. in seine Salze oder chemischen Äquivalente überführt wird.

4. Verfahren gemäß Anspruch 3, **dadurch gekennzeichnet, daß** die Fermentation unter aeroben Bedingungen bei einer Temperatur zwischen 20 und 35 °C und bei einem pH zwischen 4 und 10 durchgeführt wird.

5. Verbindungen gemäß den Ansprüchen 1 oder 2 zur Anwendung als Arzneimittel.

6. Verwendung von Verbindungen gemäß den Ansprüchen 1 oder 2 zur Herstellung von Arzneimitteln zur Behandlung von bakteriellen Erkrankungen oder Erkrankungen durch HIV.

7. Arzneimittel mit einem Gehalt an mindestens einer Verbindung gemäß den Ansprüchen 1 oder 2.

8. Verfahren zur Herstellung von Arzneimitteln gemäß Anspruch 7; **dadurch gekennzeichnet, daß** man mindestens eine Verbindung gemäß den Ansprüchen 1 oder 2 mit geeigneten Hilfs-und/oder Trägerstoffen in eine geeignete

Darreichungsform bringt.

**9.** Streptomyces species DSM 11171.

## Claims

**1.** Feglymycin, a compound of the formula:

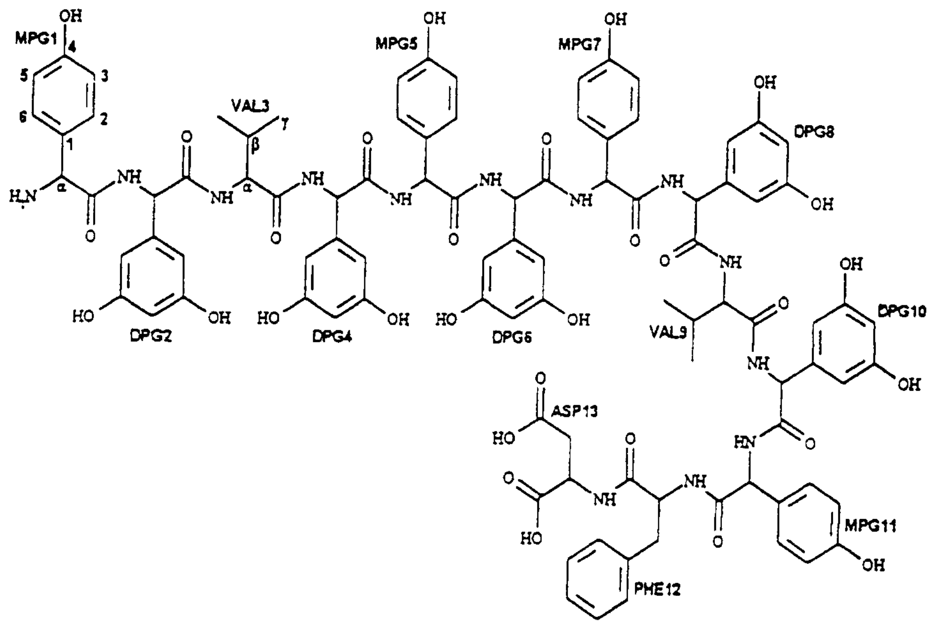

in which the abbreviations have the following meanings:

MPG = monohydroxyphenylglycine,
DPG = dihydroxyphenylglycine,
VAL = valine, PHE = phenylalanine,
ASP = aspartic acid

its physiologically tolerable salts, and its obvious chemical equivalents which exhibit a slight chemical difference with the same activity or are converted into the compound of the above formula under mild conditions.

**2.** A compound as claimed in claim 1, which can be prepared by fermenting the microorganism DSM 11171 or one of its variants or mutants under suitable conditions, isolating feglymycin and optionally converting it into its salts or chemical equivalents.

**3.** A process for the preparation of the compound as claimed in claim 1, which comprises fermenting the microorganism DSM 11171 or one of its variants or mutants under suitable conditions, isolating feglymycin and optionally converting it into its salts or chemical equivalents.

**4.** The process as claimed in claim 3, wherein the fermentation is carried out under aerobic conditions at a temperature between 20 and 35°C and at a pH between 4 and 10.

**5.** A compound as claimed in claim 1 or 2 for use as a pharmaceutical.

**6.** The use of compounds as claimed in claim 1 or 2 for the production of medicaments for the treatment of bacterial diseases or diseases due to HIV.

7. A pharmaceutical containing at least one compound as claimed in claim 1 or 2.

8. A process for the production of pharmaceuticals as claimed in claim 7, which comprises bringing at least one compound as claimed in claim 1 or 2 into a suitable administration form using suitable auxilaries and/or excipients.

9. Streptomyces species DSM 11171.

**Revendications**

1. Féglymycine, un composé de formule

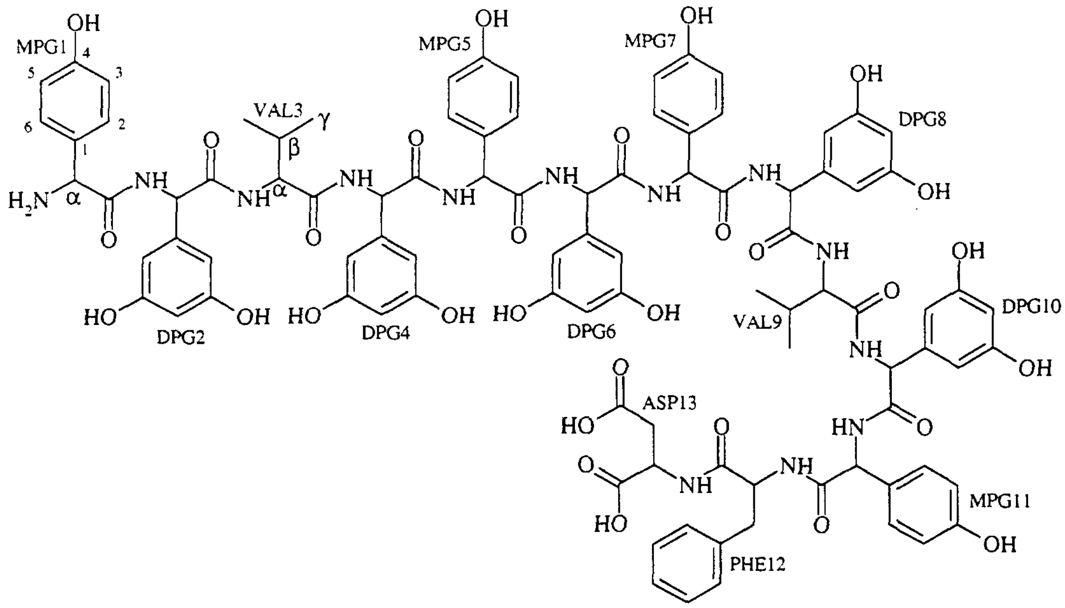

où les abréviations ont les significations suivantes :

MPG = monohydroxyphénylglycine, DPG = dihydroxyphénylglycine,
VAL = valine, PHE = phénylalanine, ASP = Acide aspartique

ses sels physiologiquement acceptables ainsi que ses équivalents chimiques manifestes, qui présentent une faible différence chimique pour la même efficacité ou qui se transforment en le composé de formule ci-dessus dans des conditions douces.

2. Composé selon la revendication 1, qui peut être préparé par le fait que le micro-organisme DSM 11171 ou l'une de ses variantes ou l'un de ses mutants est mis à fermenter dans des conditions appropriées, la féglymycine est isolée et éventuellement convertie en ses sels ou équivalents chimiques.

3. Procédé de préparation du composé selon la revendication 1, **caractérisé en ce que** le micro-organisme DSM 11171 ou l'une de ses variantes ou l'un de ses mutants est mis à fermenter dans des conditions appropriées, la féglymycine est isolée et éventuellement convertie en ses sels ou équivalents chimiques.

4. Procédé selon la revendication 3, **caractérisé en ce que** la fermentation est réalisée dans des conditions aérobies à une température située entre 20 et 35°C et à un pH situé entre 4 et 10.

5. Composés selon les revendications 1 ou 2 destinés à être utilisés comme médicaments.

6. Utilisation de composés selon les revendications 1 ou 2 pour la préparation de médicaments pour le traitement des maladies bactériennes ou des maladies dues à HIV.

7. Médicament ayant une teneur en au moins un composé selon les revendications 1 ou 2.

8. Procédé de préparation de médicaments selon la revendication 7, **caractérisé en ce que** l'on incorpore dans une forme d'administration appropriée au moins un composé selon les revendications 1 ou 2 avec des adjuvants et/ou supports appropriés.

9. Streptomyces species DSM 11171.

ULTRAVIOLETT-ABSORPTIONSSPEKTRUM DES FEGLYMYCINS AUFGENOMMEN IN METHANOL